**Europäisches Patentamt**

**European Patent Office**

**Office européen des brevets**

(19)

(11) Veröffentlichungsnummer: **0 030 316**
**A1**

(12) # EUROPÄISCHE PATENTANMELDUNG

(21) Anmeldenummer: **80107333.9**

(22) Anmeldetag: **25.11.80**

(51) Int. Cl.³: **G 02 B 25/00**, G 02 B 23/16

(30) Priorität: **05.12.79 DE 2948873**

(43) Veröffentlichungstag der Anmeldung: **17.06.81**
**Patentblatt 81/24**

(84) Benannte Vertragsstaaten: **CH FR GB LI**

(71) Anmelder: **Firma Carl Zeiss, Postfach 1369/1380, D-7082 Oberkochen (DE)**

(72) Erfinder: **Mayer, Herbert, Zähringer Strasse 6, D-7800 Freiburg (DE)**

(54) **Beschlagfreie Okulare für optische Beobachtungs- und Untersuchungsgeräte.**

(57) Okular für optische Beobachtungs- und Untersuchungsgeräte. Um ein Beschlagen des Okulars an der Einblickseite zu verhindern, ist vor der dem Auge des Benutzers zugewandten Austrittsfläche (1a) der in Lichtrichtung letzten Linse (1) des Okulars (4) ein Luftraum (7) vorgesehen, der zum Auge des Benutzers hin mit einer durchsichtigen Kunststoffscheibe (3) abgeschlossen ist. Die Wärmeleitfähigkeit dieser Kunststoffscheibe (3) ist niedriger als die der letzten Okularlinse (1).

0030316

## Beschlagfreie Okulare für optische Beobachtungs- und Untersuchungsgeräte

Die Erfindung betrifft Okulare für optische Beobachtungs- und Untersuchungsgeräte.

Bei optischen Beobachtungs- und Untersuchungsgeräten, insbesondere bei Operationsmikroskopen ist es äußerst störend, wenn sich das Okular an der Einblickseite während des Arbeitens mit Wasserdampf beschlägt. Die Intensität des Beschlagens ist vom Benutzer abhängig und umso stärker, je angestrengter dieser arbeitet.

Um diesem Übelstand abzuhelfen, liegt der Erfindung die Aufgabe zugrunde, das Beschlagen der Okulare von optischen Beobachtungs- und Untersuchungsgeräten, insbesondere von Operationsmikroskopen zu verhindern.

Diese Aufgabe wird erfindungsgemäß dadurch gelöst, daß vor der dem Auge des Benutzers zugewandten optischen Austrittsfläche der in Lichtrichtung letzten Linse des Okulars ein Luftraum vorgesehen ist, der zum Auge des Benutzers hin mit einer durchsichtigen Kunststoffscheibe abgeschlossen ist, deren Wärmeleitfähigkeit niedriger ist als die der letzten Okularlinse.

Vorteilhafterweise wird die durchsichtige Kunststoffscheibe mit Hilfe eines Distanzringes auf das Okular montiert.

Für viele Untersuchungsgeräte, besonders für Operationsmikroskope, ist es zweckmäßig, anschließend an die durchsichtige Scheibe aus Kunststoff eine Augenmuschel aus elastischem Material am Okulartubus zu befestigen. Die Befestigung kann beispielsweise mittels Schraubengewinde geschehen.

0030316

Die mit der Erfindung erzielten Vorteile bestehen insbesondere darin, daß die Lösung einfach und kostensparend ist, und daß auch vorhandene Geräte nachträglich mit der Lösung ausgerüstet werden können.

Ein Ausführungsbeispiel der Erfindung ist in der Zeichnung dargestellt und wird im folgenden näher beschrieben. Es zeigen:

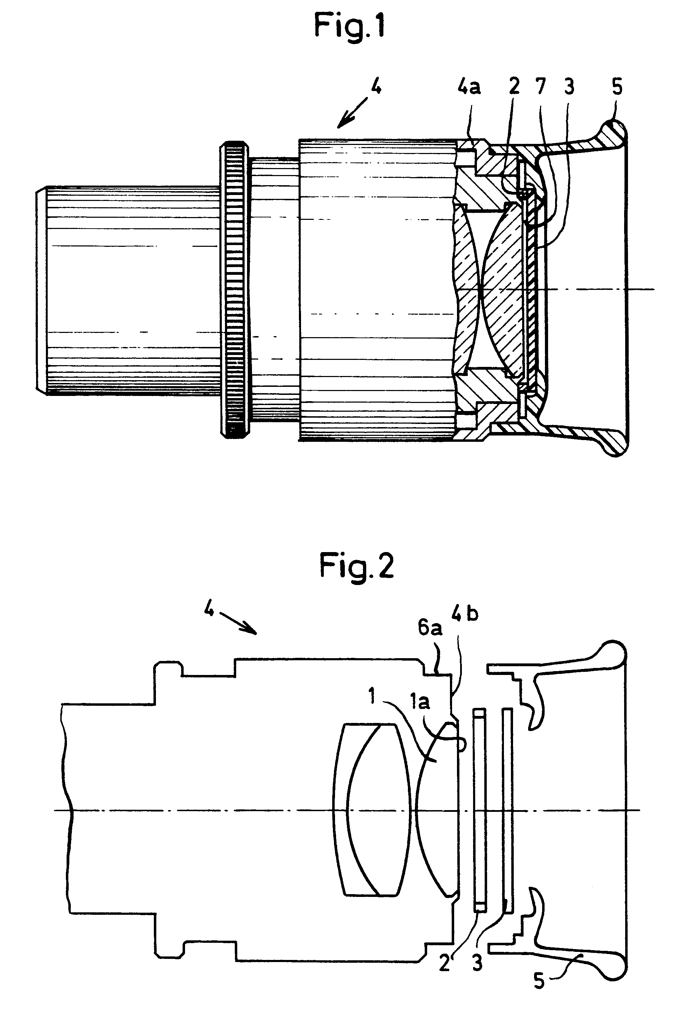

Fig. 1    einen Teilschnitt durch ein handelsübliches Okular mit der erfindungsgemäßen Anti-Beschlagsausrüstung;

Fig. 2    das in Fig. 1 gezeigte Okular in Montage-Darstellung.

Das gesamte Okular ist in der Darstellung mit 4 bezeichnet. Das Bezugszeichen 4a kennzeichnet den Okulartubus. Mit 1 ist - in Lichtrichtung gesehen - die letzte Okularlinse bezeichnet, ihre Austrittsfläche trägt die Bezeichnung 1a. Auf die äußere Ringfläche 4b des Okulartubus 4a wird ein Distanzring 2 aufgesetzt, und auf diesen die durchsichtige Kunststoffscheibe 3 gelegt. Die Gummistülpmuschel 5 ist mittels Schraubgewinde 6 und 6a auf den Okulartubus aufschraubbar und hält die Scheibe 3 in ihrer Lage. Der Luftraum 7 zwischen der letzten optischen Fläche 1a des Okulars und der Kunststoffscheibe 3 beträgt im gezeichneten Ausführungsbeispiel ca 0,2 mm. Die durchsichtige Kunststoffscheibe 3 kann beispielsweise aus Polymethylmetacrylat bestehen.

Patentansprüche

1. Okular für optische Beobachtungs- und Untersuchungsgeräte, dadurch gekennzeichnet, daß vor der dem Auge des
Benutzers zugewandten optischen Austrittsfläche (1a) der
in Lichtrichtung letzten Linse (1) des Okulars (4) ein
Luftraum (7) vorgesehen ist, der zum Auge des Benutzers
hin mit einer durchsichtigen Kunststoffscheibe (3) abgeschlossen ist, deren Wärmeleitfähigkeit niedriger ist
als die der letzten Okularlinse (1).

2. Okular nach Anspruch 1, dadurch gekennzeichnet, daß
zwischen der letzten Okularlinse (1) und der Kunststoffscheibe (3) ein Distanzring (2) vorgesehen ist.

3. Okular nach Anspruch 2, dadurch gekennzeichnet, daß am
Okulartubus (4a) eine Augenmuschel (5) aus elastischem
Material befestigbar ist.

4. Okular nach Anspruch 3, dadurch gekennzeichnet, daß die
Augenmuschel (5) an den Okulartubus (4a) mittels Gewinde
(6,6a) aufschraubbar ist.

0030316

## Fig.1

## Fig.2

| Kategorie | Kennzeichnung des Dokuments mit Angabe, soweit erforderlich, der maßgeblichen Teile | betrifft Anspruch |
|-----------|---|---|
| X | CH - A - 526 120 (LICENTIA) | 1-3 |
| | * Patentansprüche * | |
| | --- | |
| X | DE - B - 2 040 445 (LICENTIA) | 3,4 |
| | * Spalte 3, Zeilen 10-18; Figur 2 * | |
| | --------- | |

**EINSCHLÄGIGE DOKUMENTE**

**KLASSIFIKATION DER ANMELDUNG (Int. Cl.³)**

G 02 B 25/00
G 02 B 23/16

**RECHERCHIERTE SACHGEBIETE (Int. Cl.³)**

G 02 B 23/16
G 02 B 25/00
A 61 B 1/24

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie. übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 10-03-1981 | PFAHLER |

EPA form 1503.1  06.78

BAD ORIGINAL